Europäisches Patentamt

European Patent Office

Office européen des brevets

(11) Veröffentlichungsnummer: **0 425 686 A1**

# (12) EUROPÄISCHE PATENTANMELDUNG
## veröffentlicht nach Art. 158 Abs. 3 EPÜ

(21) Anmeldenummer: **90905355.5**

(22) Anmeldetag: **28.09.89**

(86) Internationale Anmeldenummer:
**PCT/SU89/00259**

(87) Internationale Veröffentlichungsnummer:
**WO 90/14120 (29.11.90 90/27)**

(51) Int. Cl.5: **A61M 5/50**

(30) Priorität: **16.05.89 SU 4684319**

(43) Veröffentlichungstag der Anmeldung:
**08.05.91 Patentblatt 91/19**

(84) Benannte Vertragsstaaten:
**BE CH DE FR GB IT LI SE**

(71) Anmelder: **POLTAVSKY MEDITSINSKY STOMATOLOGICHESKY INSTITUT**
**ul. Shevchenko, 23,**
**Poltava 314000(SU)**

(72) Erfinder: **MAZURIK, Sergei Mikhailovich**
**ul. Lenina, 92-57**
**Poltava, 314022(SU)**
Erfinder: **EFREMOV, Oleg Viktorovich**
**ul. 60 let Oktyabrya, 3-58 Poltavskaya obl.**
**Karlovka, 315720(SU)**

(74) Vertreter: **Sparing Röhl Henseler**
**Patentanwälte European Patent Attorneys**
**Rethelstrasse 123**
**W-4000 Düsseldorf 1(DE)**

(54) **EINMALIGE SPRITZE FÜR INJEKTIONEN.**

(57) Einmalige Spritze für Injektionen enthält eine Zylinderflasche (I), in dieser untergebrachten Kolben (5) mit seiner Stange (6) und eine Fassung (4) zum Festhalten der Nadel. Der Kolben (5) und seine Stange (6) stehen in keiner mechanischen Verbindung miteinander, während die Kolbenstange (6) an ihrem Kolbenseitigen Ende einen Ringbund (7) aufweist und eine an der Kolbenstange (6) koaxial aufgesetzte Scheibe (8) trägt. Der Durchmesser der Bohrung der Scheibe (8) ist kleiner als der Durchmesser des Ringbundes (7), während der Aussendurchmesser der Scheibe (8) den Innendurchmesser der Zylinderflasche (I) so weit übersteigt, dass die Reibungskraft zwischen der Aussenmantelfläche der Scheibe (8) und der Innenmantelfläche der Zylinderflasche (I) die an der Scheibe (8) bei den Bewegungsgängen der Zylinderflasche (I), darunter auch mit Beschleunigung angreifende Kraftbelastung übertrifft.

Erfindungsgemässe einmalige Spritze kann sowohl in jeden medizinischen Anstalten als auch bei dem individuellen Gebrauch ihre Anwendung finden.

FIG.5

EP 0 425 686 A1

## EINMALIGE SPRITZE FUR INJEKTIONEN

Technisches Gebiet

Die Erfindung bezieht sich auf das Gebiet medizinische Ausrüstungen, insbesondere auf einmalige. bei den Injektionen ihre Anwendung findende Spritzen.

Zugrundliegender Stand der Technik

Zur Zeit sind allgemein bekannt die einmaligen Spritzen für Injektionen. die einen Zylinderflasche, einen in dieser untergebrachten Kolben mit seiner Stange und eine Vorrichtung zum Festhalten der Nadel wie beispielsweise die einmaligen Spritzen der Firma "Trumo Europe" Produktion. Belgien einschliessen. Die betreffenden Spritzen unter scheiden sich in ihrem Aufbau von den weitgehend bekannten, in der medizinischen Praxis erweiterte Anwendung findenden, mehrmaligen Spritzen praktisch in nichts unter der Ausnahme aber, dass sie aus einem billigeren polymeren Werkstoff hergestellt sind und keiner Sterilisation unterliegen. Mit anderen Worten besteht die Möglichkeit, durch den Aufbau der einmaligen Spritzen diese mehrmals auszunutzen. Diese mehrmalige Benutzung kann infolge fehlender Aufmerksamkeit und keiner Gewissenhaftigkeit der medizinischen Fachkraft. bei der Durchführung der Injektionen von narkotisch- oder alkoholischbesoffenen Personen zustande kommen. Alles gesagte wäre als äusserstwichtig zu betrachten, besonders wenn unter diesen Umständen eine Ansteckung mit dem AIDS-Virus, mit dem AIDS-VIRUS der Infektionshepatitis und anderer Erkrankungen durchaus möglich ist.

Bekannt ist auch eine andere einmalige Spritze, die ebenfallse ihre Zylinderflasche, in dieser untergebrachten Kolben mit seiner Stange und eine Fassung zum Festhalten der Nadel vorsieht (EP. A, 0282097).

In dieser Spritze ist die Nadel in einer im Vorderteil der Zylinderflasche der Spritze eingebauten, an deren Achse entlang hin- und hervorstellbaren Scheibe festgelegt. Der hinter dieser Scheibe im Inneren der Zylinderflasche der Spritze untergebrachte, mit seiner Stange starrverbundene Kolben steht mit der Scheibe in keiner Verbindung. weist aber die Zusammenkoppelung des Kolbens mit der Scheibe ausgerichtete Greifer auf, welche Zusammenkoppelung bei Inkontakttreten deren Stirnflanken zustandekommt. Am Ende der durchgeführten Injektion greift der Kolben mit seiner Greifern bei der Berührung der Stirnflanken der Scheibe und des Kolbens in die Scheibe starr untrennbar an, in der die Nadel festeingeklemmt bleibt: infolgedessen wird die Scheibe bei einer wiederholten Einsaugung des Injektionsmittels in die Spritze hinter dem Kolben ins Innere der Zylinderflasche der Spritze miteingezogen. Bei diesem Hineinziehen der Nadel ins Innere der Zylinderflasche erfolgt deren Versetzung bezüglich der Achse der Zylinderflasche. Der Versuch, eine wiederholte Injektion durchzuführen, führt zu der Zerstörung der Injektionsnadel.

Trotzdem bleibt auch bei diesem Aufbau der Spritze eine Möglichkeit vorhanden, die Spritze wiederholt zu benutzen. da der Kolben mit der Scheibe nur in seiner Endstellung im Vorderteil der Zylinderflasche in den untrennbaren Eingriff tritt. Nur unter diesem Umstand erfolgt das Einziehen der Nadel in die Zylinderflasche hinein und die Spritze fällt aus ihrer weiteren Benutzung aus. Bleibt aber der Kolben noch vor dessen Endstellung bei der Durchführung der jeweiligen Injektion nicht bis zum Ende hineingeschoben, so kann die Spritze bei der beliebig grossen Anzahl der Injektionen unter fast vollkommener Ausnutzung des ganzen Rauminhaltes deren Zylinderflasche gebraucht werden.

Offenbarung der Erfindung

Der vorliegenden Erfindung liegt die Aufgabe zugrunde. eine aufbaumässige Ausführung der einmaligen Spritze zu entwickeln, durch welche jede Möglichkeit die Spritze wiederholt auszunutzen ausgeschlossen wird.

Das Wesen der vorliegenden Erfindung besteht darin, dass in der einmaligen Spritze, die ihre Zylinderflasche, in dieser untergebrachten Kolben mit seiner Stange und eine Fassung zum Festhalten der Nadel vorsieht, erfindungsgemäss der Kolben mit seiner Stange in keiner mechanischen Verbindung steht, die Kolbenstange einen kleineren Durchmesser als der Durchmesser des Kolbens aufweist und an ihrem kolbenseitigen Ende einen Ringbund und eine daran koaxial angebrachte Scheibe trägt, deren Durchmesser kleiner als dieser des Ringbundes ist und der Aussendurchmesser den Innendurchmesser der Zylinderflasche so weist übertrifft, dass die Reibungskraft zwischen der Aussenmantelfläche der Scheibe und der Innenmantelfläche der Zylinderflasche die an der Scheibe bei den Bewegungsgängen der Zylinderflasche, darunter auch mit der Beschleunigung angreifende Kraft übersteigt.

Die einmalige erfindunsgemässe Spritze lässt jede Môglichkeit ausschliessen, eine weiderholte Durchführung der Injektion zu verwirklichen, ist in ihrer Herstellung einfach und im Gebrauch sicher. Die Produktionskosten dieser einmaligen Spritze übersteigen diese bei der Fertigstellung der bisher bekannten einmaligen Spritzen praktisch in nichts.

Kurzbeschreibung der Zeichnungen

Nachstehend wird die Erfindung an Hand der Reschreibung deren Ausführungsbeispiels unter Rezugnahme auf angelegte Zeichnungen näher erläutert, in denen es zeigt:
Fig. I, 2, 3, 4 erfindungsgemässe einmalige Spritze Für Injektionen, auseinandergestellt in räumlicher Darstellung;
Fig. 5 erfindungsgemässe einmalige Spritze für Injektionen, zusammengestellt in ihrer Ausgangsstellung vor dem Einsaugen des Injektionsmittels, Längsschnitt;
Fig. 6 dieselbe aus der Fig. 5 beim Einsaugen des Injektionsmittels;
Fig. 7 dieselbe aus der Fig. 5 nach der Einführung des Injektionsmittels;
Fig. 8 dieselbe aus der Fig. 5 beim Versuch, wiederholt das Injektionsmittel einzusaugen.

Beste Ausführungsvariante der Erfindung

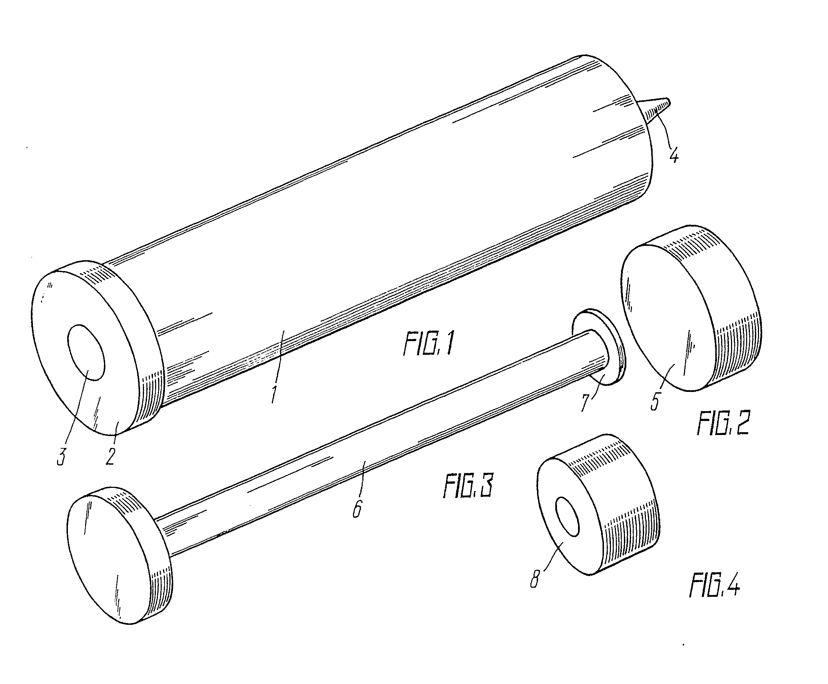

Erfindungsgemäss ausgeführte einmalige Spritze für Injektionen enthält eine Zylinderflasche I (Fig. I, 5). an deren einer Stirnflanke ein mit der Zylinderflasche I untrennbar verbundener Deckel 2 festgesetzt ist. In diesem Deckel 2 ist eine Zentralbohrung 3 ausgespart. An der anderen Stirnflanke der Zylinderflasche I ist eine Fassung zum Festhalten der Nadel. die in Form einer Kanüle 4 ausgeführt ist. Innerhalb der Zylinderflasche I ist ein Kolben 5 (Fig. 2, 5 bis 8) untergebracht, der den Querschnitt der Zylinderflasche I dicht verschlägt.

Hinter dem Kolben 5 ist eine Kolbenstange 6 (Fig. 3, 5 bis 8) innerhalb der Zylinderflasche I eingeschoben. Diese Kolbenstange 6 steht mit dem Kolben 5 in keiner mechanischen Verbindung und weist einen kleineren Durchmesser als bei dem Kolben 5 und der Innenbohrung 3 auf, so dass eine freie Bewegung der Kolbenstange 6 im Zylinderflasche I über die Zentralbohrung 3 im Deckel 2 erreicht wird. An ihrem kolbenseitigen Ende trägt die Kolbenstange 6 einen Ringbund 7 und eine daran koaxial aufgesetzte Scheibe 8 (Fig. 4 bis 8).

Der Durchmesser der Zentralbohrung der betreffenden Scheibe 8 übertrifft den Durchmesser der Kolbenstange 6 und weist einen kleineren Durchmesser als der des Ringbundes 7. Der Aussendurchmesser der Scheibe 8 übertrifft auch den Innendurchmesser der Zylinderflasche I so weit.

dass die Reibungskraft zwischen der Aussenmantelfläche der Scheibe 8 und der Innenmantelfläche der Zylinderflasche I der an der Scheibe 8 bei den Bewegungsgängen der Zylinderflasche I, darunter auch mit Beschleunigung angreifenden Kraft wesentlich überlegen ist.

Die erfindungsgemässe einmalige Spritze für Jnjektionen wird wie folgt gebraucht.

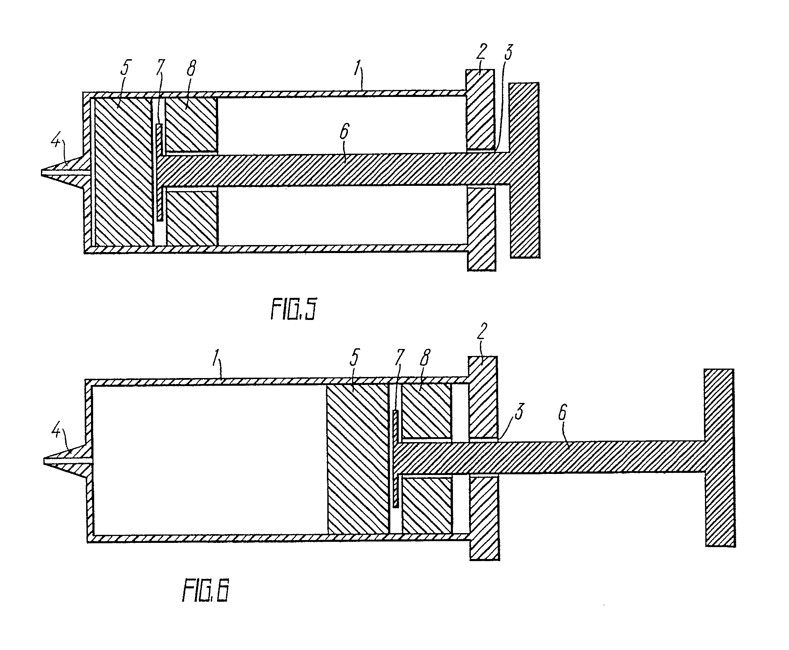

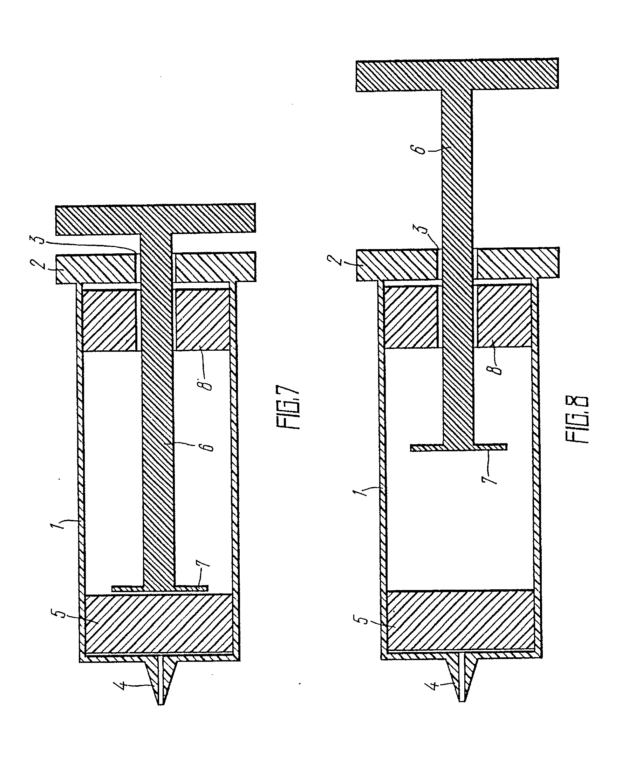

In seiner Ausgangsstellung befindet sich der Kolben 5 an dem Vorderstirnteil der Zylinderflasche I der Spritze, der die Kanüle 4 trägt (Fig. 5). In dieser Stellungslage liegt die Kolbenstange 6 an der Ebene ihres Ringbundes 7 an dem Kolben 5 an. Dabei stösst die Scheibe 8 gegen den Ringbund 7 auch. Zum Einsaugen des jeweiligen Injektionsmittels wird die Kolbenstange 6 über die Zentralbohrung 3 aus der Zylinderflasche I der Spritze herausgezogen. Dabei drückt sich der Ringbund 7 der Kolbenstange 6 an der Scheibe 8 dicht heran. indem dadurch eine hermetische Abdichtung des Hubraumes zwischen der Kolbenstange 6 mit der Scheibe 8 einerseits und dem Kolben 5 andererseits zustandekommt durch welche die Hubbewegung des Kolbens 5 hinter der Kolbenstange 6 und der Scheibe 8 beim ganzen Bewegungsgang der Kolbenstange 6 beim Einsaugen des Injektionsmittels (Fig. 6) erreicht wird. Bei der Injektion wird die Kolbenstange 6 rückwärts in die Zylinderflasche I der Spritze hineingeschoben und dadurch auch der Kolben 5 (Fig. 7) zu der Vorderflanke der Zylinderflasche I zurückverstellt. Die Scheibe 8 verbleibt aber an dem Hinterstirnteil der Zylinderflasche I der Spritze, an dem Deckel 2 neben. was die Reibungskraft zwischen der Aussenmantelfläche der Scheibe 8 und der Innenmantelfläche der Zylinderflasche I der spritze besorgt. Der Aussendurchmesser der Scheibe 8 wird derart ausgewählt, dass die Reibungskraft zwischen der Aussenmantelfläche der Scheibe 8 und der Innenmantelfläche der Zylinderflasche I der Spritze im wesentlichen die an der Scheibe 8 bei den axialen Bewegungsgängen der Zylinderflasche I selbst auch mit der Beschleunigung angelegte Kraftbelastung übersteigt, wodurch jede Möglichkeit entfällt, die Scheibe 8 in ihre Ausgangsstellung auch der Zurückschiebung des Kolbens 5 zu der Vorderstirnflanke der Zylinderflasche I der Spritze zurückzuverstellen. Eim mechanischer Rückschub der Scheibe 8 in ihre Ausgangsstellung ist ebenfalls unmöglich, da der Deckel 2 an der Zylinderflasche I untrennbar festgesetzt ist.

Ein Versuch, die wiederholte Einsaugung des Mittels für die Injektion in die Spritze durchzuführen, misslingt, solange die Kolbenstange 6 ohne die Scheibe 8 keinenfalls eine hermetische Abdichtung und Unterdruck innerhalb des Zwischenhubraumes entwickeln kann, die für die Verstellung des Kolbens 5 notwendig sind (Fig. 8).

Dadurch wird nur die einmalige Benutzung der erfindungsgemässen Spritze erreicht, die dann einer Verwertung unterliegt.

Eine weitgehende Anwendung der erfindungsgemässen einmaligen Spritze kann jeweils die Ansteckung des Patienten bei der Injektion mit dem AIDS-Virus, mit dem Virus der Infektionshepatitis und anderer Erkrankungen praktisch ausgeschlossen, die bei der parenteralen Einführung der Heilmittel übertragen werden.

Industrielle Verwertbarkeit

Die Erfindung kann für die Durchführung der subkutanen,intramuskulären und intravaskulären Infektionen eingesetzt werden und kann in beliebigen medizinischen Anstalten sowie beim individuellen Gebrauch in Frage kommen.

**Ansprüche**

Einmalige Spritze für Injektionen, die eine Zylinderflasche (I), in dieser untergebrachten Kolben (5) mit seiner Stange (6) und eine Fassung zum Festhalten der Nadel vorsieht, dadurch **gekennzeichnet,** dass der Kolben (5) und seine Stange (6) in keiner mechanischen Verbindung miteinander stehen, die Kolbenstange (6) einen kleineren Durchmesser als der Durchmesser des Kolbens (5) aufweist und an ihrem Ende seitens des Kolbens (5) einen Ringbund (7) und eine an der Kolbenstange (6) koaxial aufgesetzte Scheibe (8) trägt, deren Innendurchmesser in der Zentralbohrung kleiner als der Durchmesser des Ringbundes (7) ist, während der Aussendurchmesser den Innendurchmesser der Zylinderflasche (I) so weit übersteigt, dass die Reibungskraft zwischen der Aussenmantelfläche der Scheibe (8) und der Innenmantelfläche der Zylinderflasche (I) die an der Scheibe (8) bei den Bewegungsgängen der Zylinderflasche (I), darunter auch mit der Beschleunigung angreifende Kraftbelastung übertrifft.

FIG.1

FIG.2

FIG.3

FIG.4

EP 0 425 686 A1

FIG.5

FIG.6

FIG.7

FIG.8

EP 0 425 686 A1

# INTERNATIONAL SEARCH REPORT

International Application No **PCT/SU 89/00259**

**I. CLASSIFICATION OF SUBJECT MATTER** (if several classification symbols apply, indicate all) *

According to International Patent Classification (IPC) or to both National Classification and IPC

IPC5:    A 61 M 5/50

**II. FIELDS SEARCHED**

Minimum Documentation Searched 7

| Classification System | Classification Symbols |
|---|---|
| IPC4: | A 61 M 5/00, 5/18, 5/24, 5/315 |

Documentation Searched other than Minimum Documentation
to the Extent that such Documents are Included in the Fields Searched 8

**III. DOCUMENTS CONSIDERED TO BE RELEVANT** 9

| Category * | Citation of Document, 11 with indication, where appropriate, of the relevant passages 12 | Relevant to Claim No. 13 |
|---|---|---|
| A | SU, A3, 1082305 (TULSEA S.A.), 23 March 1984 see the abstract | 1 |
| A | GB, A, 2205750 (KENNETH GEORGE HUNT et al.), 21 December 1988, see the abstract | 1 |
| A,P | EP, A3, 0329358 (R&R INVENTIONS LIMITED), 23 August 1989, see the abstract | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 12 June 1990 (12.06.90) | 2 July 1990 (02.07.90) |
| International Searching Authority | Signature of Authorized Officer |
| ISA/SU | |

Form PCT/ISA/210 (second sheet) (January 1985)